# EUROPEAN PATENT SPECIFICATION

(11) **EP 2 086 595 B1**
(45) Date of publication and mention of the grant of the patent: **19.08.2015**
(21) Application number: 07847168.7
(22) Date of filing: 14.11.2007
(51) Int. Cl.: B01J 8/10, B09B 3/00, A61L 11/00

(54) **PROCESS AND APPARATUS FOR WASTE TREATMENT**
VERFAHREN UND VORRICHTUNG ZUR ABFALLBEHANDLUNG
PROCÉDÉ ET APPAREIL POUR LE TRAITEMENT DES DÉCHETS

(30) Priority: 27.11.2006 ES 200603022
(43) Date of publication of application: 12.08.2009
(73) Proprietor: Ambiensys, S.L., 08213 Polinya (ES)
(72) Inventor: RIBAS LOPEZ, Rafael, 08210 Barbera Del Valles (ES); RIBAS MARTINEZ, Oscar, 08210 Barbera del Valles (ES)
(74) Representative: Sugrañes Patentes y Marcas
(86) International application number: PCT/EP2007/062353
(87) International publication number: WO 2008/065002

(56) References cited:
- EP-A- 0 870 433
- WO-A-02/085796
- WO-A-03/024633
- WO-A-2006/121697
- DE-A1- 4 109 829
- US-A1- 2003 147 771
- US-A1- 2006 222 574

## Description

### Technical field of the invention

The present invention relates to a process according to claim 1 and an apparatus according to claim 9.

### Background of the invention

Among the different known processes for the treatment of urban solid waste or household waste, as well as similar industrial waste, that where the waste is sterilized and partially desiccated is of particular interest, transforming the organic fraction of the waste in a homogenized by-product for its subsequent separation and use via recycling circuits and/or energy recovery.

In these types of processes, also called waste sanitation, the waste is exposed during a predetermined period of time to a steam bath, at a high temperature, at the same time as being mechanically agitated with the aim, among others, of extracting the liquids the waste contains.

The apparatuses especially designed to carry out this sanitation process generally comprise a rotary drum of large dimensions, conventionally of cylindrical shape, capable of rotating around its longitudinal axis. These receptacles are provided with an opening, provided with a gate which allows the receptacle to be hermetically closed, wherein the waste to be treated is introduced and the waste is extracted therefrom once treated.

The process followed for the sanitation of the waste first consists of introducing a large quantity of waste in the receptacle, hermetically closing the receptacle and then increasing the pressure and temperature therein whilst the waste is agitated as the receptacle rotates around its longitudinal axis. When the pressure and temperature inside the receptacle reach the desired values, the pressure and temperature conditions inside the receptacle are maintained constant for a predetermined time, which varies in accordance with the composition of the waste, producing the actual waste sanitation. Then the pressure is decreased inside the receptacle in controlled manner and the waste is extracted therefrom.

As a representative part of the state of the art, documents US 5427650, WO 03025101 and US 5427650 are cited, which disclose sanitation processes that follow the aforementioned steps as well as apparatuses especially suitable for performing said processes.

One of the greatest drawbacks of the sanitation processes disclosed in said documents is related to the high energy consumption necessary, in each operating cycle, to raise the pressure and temperature inside the receptacle after the introduction of the waste therein, generally introduced at atmospheric pressure and at ambient temperature, until the necessary values for its sanitation, and the consequent energy loss produced later when, once the waste is treated for a predetermined time at the working conditions, the receptacle is depressurized to safely open its gate and extract the waste from inside it. Naturally, the opening of the receptacle also entails a considerable lowering of the temperature inside the receptacle. In a following operative cycle, after introducing a new quantity of waste inside the receptacle sufficient new energy should be provided to raise the pressure and the temperature inside the receptacle to the suitable values for waste sanitation.

The fact that the pressure and temperature inside the receptacle have to be increased to the suitable values for the waste treatment in each operative cycle, in addition to the energy cost it entails for the production of the necessary water vapour, appreciably extends the duration of each operative cycle.

EP0870433 refers to a process and a device for inactivating industrial waste according to the preamble of method claim 1 and apparatus claim 9.

WO 02/085796 discloses a method and a device for treating feedstocks, that include large solid objects, dissolved and undissolved solids, sludges or slurries that contains organics that may be volatilized and then oxidized or reformed hydrothermally.

DE 41 09 829 discloses a method and a device for decontaminating infectious waste in a continuous mode or in a batch mode.

US 2006/222574 refers to a method and a device for the digestion and sanitary disposal of waste material, such as infectious waste material and other hazardous, biohazardous, or radioactive waste, placing said waste in a permeable container, such as a basket to completely immerse the waste material within a solvent solution in batches.

WO 03/024633 refers to a process and a device for treating urban solid waste, in which urban solid waste is entered into a rotating autoclave and moved forward and backward while being treated in batches.

WO 2006/121697 refers to a process and a device for treating urban solid waste, in which urban solid waste is entered into a rotating vessel and moved from a first end to a second end while being treated in batches.

At this point we should state that the great quantity of waste generated in the large population centres obliges increasing the dimensions of the receptacles designed to treat the waste, since the treatment facilities should have sufficient capacity to treat a high volume of waste at the same time. Nevertheless, the greater dimension of the receptacle the greater the energy consumption necessary and the time required to reach the suitable pressure arid temperature conditions inside the receptacle, for the waste sanitation, each time an operative cycle starts.

A first objective of the invention is to disclose a process and an apparatus which improve the output of the known processes and apparatuses, reducing the energy consumption and the time necessary per unit of volume of treated waste.

On the other hand, and in relation to the known apparatuses, the use as pressure tanks of the rotary pressure receptacles involves the use of suitable materials to resist the mechanical stresses or demands to which these receptacles are subjected due to being rotary, and also to resist the high pressure and temperatures inside the receptacles. Furthermore, the inner walls of the receptacles, which are in direct contact with the waste, should resist the mechanical aggressions to which the solid waste is subjected, on being agitated, they continually impact and rub against the inner walls of the receptacles.

In addition to the aforementioned drawbacks, regarding the mechanical stresses and inner wear to which these rotary receptacles are subjected, the receptacles described in said documents have other drawbacks related to the complexity of the communications between the inside and outside of the receptacle, especially regarding the passages for the intake of steam and for the outlet of liquids, if we do not want to alter the pressure and temperature conditions inside the receptacle and hinder the rotary movement to which it is subjected.

It is therefore another object of the invention to disclose an apparatus for waste treatment which is simpler and/or reduces the manufacturing costs in comparison with known apparatuses.

It is also an objective of the apparatus according to the invention to improve the extract released by the waste inside the receptacles, consequently improving the drying level and the quality of the waste obtained, in the sense that its water content is reduced in comparison with the waste obtained from carrying out the sanitation process in a conventional apparatus.

### Explanation of the invention

The process for waste treatment object of the invention is particularly suitable for the treatment of urban solid waste and similar industrial waste, and is characterized in that it comprises the continuous operation of treating the waste in a pressure tank, turning it and/or mixing it and subjecting it, in an uninterrupted manner, during its retention in the closed enclosure to a pressure over 2 bar and a temperature between 100 °C and 200 °C, and the discontinuous operations of subjecting to pressure, one by one, successive batches of loaded waste and introducing them, also one by one, inside the pressure tank; extracting from the pressure tank and decompressing, also one by one, several already treated batches of discharged waste, performing the corresponding compression and decompression operations in respective loading or compression chambers and discharge or decompression chambers, wherein the pressure to which the waste is subjected is increased from ambient pressure to that of inside the pressure tank and vice-versa, respectively, compensating the small pressure and temperature losses inside the pressure tank whose values are maintained essentially constant, after the waste introduction and extraction operations.

Unlike the known processes, the pressure tank is not depressurized every time waste is extracted from inside it nor is it necessary to increase the pressure therein, from atmospheric pressure until reaching a pressure over 2 bar, each time new waste to be treated is introduced.

The waste from multiple batches of loaded waste successively introduced therein accumulate in the pressure tank.

According to another characteristic of the invention, there is a pressure tank, inside which the waste is made to pass through a rotary drum, coaxial with the pressure tank, of perforated walls through which the liquids may flow towards the exterior of the rotary drum.

Preferably, the perforations are of aproximately 1 mm to aproximately 7 mm of diameter.

In accordance with another characteristic of the process according to the invention, a communication is established between said loading or compression and discharge or decompression chambers designed to equal the pressures in both chambers at least during the compression and decompression operations.

Before the introduction operation of a batch of loading waste in the loading or compression chamber, the waste of said batch of loading waste may be sufficiently compacted to deform or break elements that exceed predetermined dimensions or to detect the inclusion of unbreakable elements that exceed said predetermined dimensions, avoiding the unnecessary dripping of liquids contained in the waste, to authorize the introduction of the batch of loading waste in the loading or compression chamber, and subsequently in the pressure tank, or temporarily reject it.

According to the invention, an apparatus for treatment, of urban solid waste and similar industrial waste is disclosed, equipped with a fixed pressure tank being arranged at one longitudinal end an inlet of the waste to treat and at the other longitudinal end, an outlet of the treated waste, being disposed inside the pressure tank means to transport the waste introduced from the inlet to the outlet of the pressure tank and turn and/or agitate said waste.

In essence, the apparatus object of the invention comprises a loading or compression chamber, designed to house a batch of loading waste to treat, equipped with separate inlet and outlet channels of the waste to which the outlet channel gives access, through the inlet of the pressure tank, to the inside of said pressure tank, both inlet and outlet channels being equipped with respective sluice gates; a discharge or decompression chamber, designed to house a batch of discharged waste, equipped with inlet and outlet channels of the already treated waste, of which the inlet channel is juxtaposed to the pressure tank outlet, both inlet and outlet channels being provided with respective sluice gates; and means to increase and decrease the pressure inside the loading or compression and discharge or decompression chambers from atmospheric pressure until reaching the value of the pressure inside the pressure tank and vice-versa, respectively.

According to another characteristic of the invention, the means to transport, turn and/or agitate the waste inside the pressure tank comprise a rotary drum, coaxial respect to the pressure tank, of perforated walls and equipped with elements which are projected from the inner surface of the side wall of said rotary drum, designed to turn and to move in longitudinal direction, respectively, the waste introduced therein with the rotation of the rotary drum around its longitudinal axis.

Preferably, the diameter of the perforations are of aproximately 1 mm to aprox. 7 mm., in order to prevent the waste from flowing towards the exterior of the tubular body but enabling the liquids released by the waste to flow through said perforations.

In accordance with another characteristic of the invention, the walls of the rotary drum are only perforated along an end longitudinal portion of said rotary drum, corresponding to the reception end of the waste.

In accordance with another characteristic of the invention, the outlet channel of the loading or compression chamber is disposed inside the pressure tank so that when the waste contained in said loading or compression chamber is extracted, it is deposited, by gravity, in the means to transport, turn and/or agitate the waste.

According to another characteristic of the invention, the loading or compression chamber of the waste is essentially cylindrical, is disposed horizontally and is equipped with means of pushing the waste contained in direction of the outlet channel.

In accordance with another characteristic of the invention, the means of pushing comprises a hydraulic cylinder which can move longitudinally through the inside of the loading or compression chamber.

In accordance with another characteristic of the invention, the outlet of the pressure tank is disposed in the lower side thereof and the inlet channel of the discharge or decompression chamber is disposed juxtaposed under said outlet, so that the waste extracted from the pressure tank is introduced, by gravity, in said discharge or decompression chamber.

According to another characteristic of the invention, the apparatus is equipped with means of communication between the loading and compression and discharge or decompression chambers, whereby the internal pressures of both chambers can be equalled.

According to a particularly interesting embodiment, the pressure tank is disposed inclined with respect to the horizontal and so that the end equipped with the outlet is raised with respect to the end equipped with the inlet.

### Brief description of the drawings

The attached drawings illustrate, by way of non-limitative example, a preferred embodiment of the apparatus for waste treatment object of the invention. In said drawings:
- Fig. 1: is a longitudinal section view of the apparatus for waste treatment; and
- Figs. 2, 3 and 4: are schematic views of the apparatus of Fig. 1 in successive stages of the waste treatment process object of the invention.

### Detailed description of the drawings

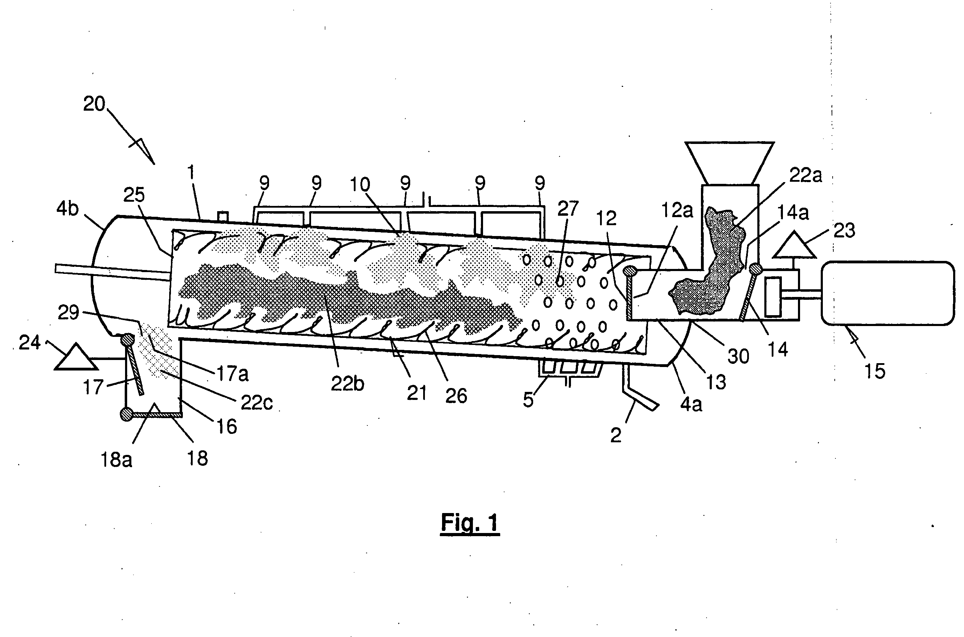

The apparatus 20 represented in Figs. 1 to 4 is designed for treatment of urban solid waste and similar industrial waste such as cardboard, office waste and derivatives of industrial processes such as waste from slaughter houses, vegetable plantations, composting plants, etc.

In Fig. 1 it is observed that said apparatus 20 is equipped with a fixed pressure tank 1 at whose longitudinal ends 4a and 4b are disposed separate inlets 30 of the waste to treat and outlets 29 of the treated waste and inside which the waste 22b is subjected during a predetermined period of time to determined pressure and temperature conditions in accordance with its composition. Means to transport 21, turn and/or agitate the waste introduced from the inlet 30 to the outlet 29 are disposed inside the pressure tank 1. The apparatus 20 also comprises a loading or compression chamber 13, a discharge or decompression chamber 16 and means 23 and 24 to increase the pressure inside said chambers from atmospheric pressure until reaching the pressure value inside the pressure tank 1 and vice-versa.

It is convenient to mention that the apparatus 20 operates in continuous process, which means that the waste to treat is introduced inside the pressure tank 1 by the inlet 30 discontinuously, in successive batches of loaded waste 22a, without interrupting the treatment of the waste 22b which is carried out inside the pressure tank 1, and the treated waste 22b is extracted from inside the pressure tank 1 in successive batches of discharged waste 22c through the outlet 29, also without interrupting the treatment of the waste 22b which continues inside the pressure tank 1. In other words, the operations of introducing and extracting batches of loaded waste 22a and batches of discharged waste 22c from inside the pressure tank 1 are simultaneous with the waste 22b treatment operation which is carried out in continuous and uninterrupted form whilst there is waste inside the pressure tank 1.

With regard to the pressure tank 1, this preferably has the capacity for housing the content of multiple batches of loaded waste 22a successively introduced in said pressure tank 1, which are accumulated therein and are turned and/or mixed in uninterrupted manner, subjected to a pressure over 2 bar and a temperature between 100 °C and 200 °C throughout its retention inside the pressure tank 1. For said purpose, the pressure tank 1 is equipped with a series of intake conduits 9 of gas, and preferably steam, whereby this is injected inside the pressure tank 1 to maintain the suitable pressure and temperature conditions for the correct waste 22b sanitation.

Due to the fact that inside the pressure tank 1 there is a pressure higher than the outside pressure or atmospheric pressure at which the batch of loaded waste 22a to treat is subjected, the apparatus 20 comprises a loading or compression chamber 13 to compensate this pressure difference and thus be able to introduce the waste inside the pressure tank 1 without significantly altering the pressure conditions inside the pressure tank 1.

The loading or compression chamber 13, essentially cylindrical and horizontally disposed, is designed to house a batch of loaded waste 22a of waste to treat, equipped with separate waste inlet 14a and outlet 12a channels. The outlet channel 12a gives access, through the inlet 30 of the pressure tank 1, inside said tank, both inlet 14a and outlet channels 12a being provided with respective sluice gates 14 and 12.

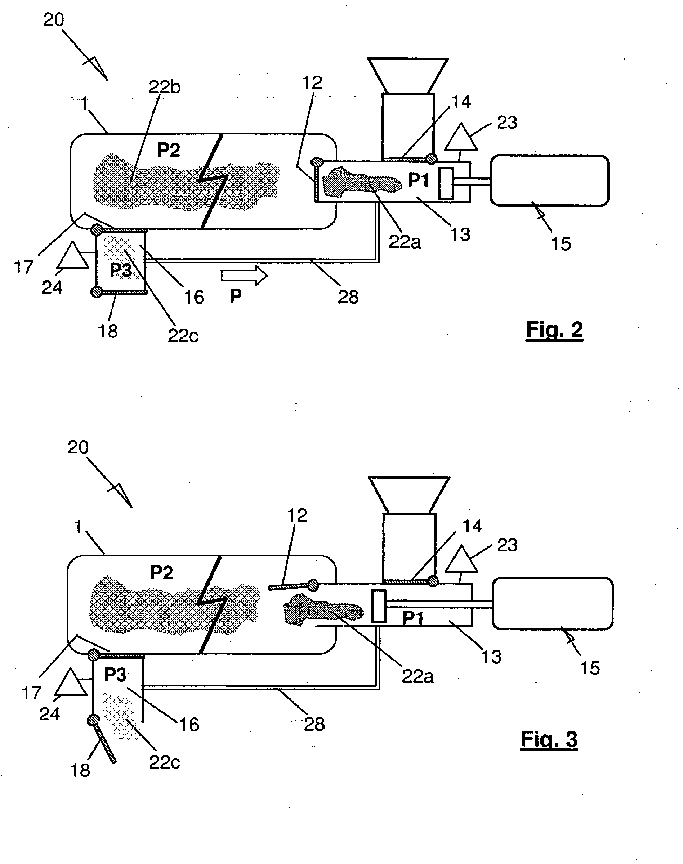

To introduce a batch of loaded waste 22a to treat inside the pressure tank 1 the following is done. With the sluice gate 12 of the loading or compression chamber 13 closed, a batch of loaded waste 22a is introduced through its inlet channel 14a (situation represented in Fig. 1). Next, the sluice gate 14 is closed and the pressure P1 is increased in the loading or compression chamber 13 by means 23, which inject steam therein, until reaching the pressure P2 value inside the pressure tank 1. Once the pressures P1 and P2 (situation represented in Fig. 3) are equalled the sluice gate 12 of the loading or compression chamber 13 opens and the batch of loaded waste 22a is pushed by means of pushing 15, formed by a hydraulic cylinder which can move longitudinally through the inside of the loading or compression chamber 13, to the inside of the pressure tank 1.

It is observed in Figs. 1 to 3 that the outlet channel 12a of the loading or compression chamber 13 is disposed inside the pressure tank 1 so that, when the batch of loaded waste 22a contained in said loading or compression chamber 13 is extracted, it is deposited, by gravity, in the means to transport 21, turn and/or agitate the waste 22b.

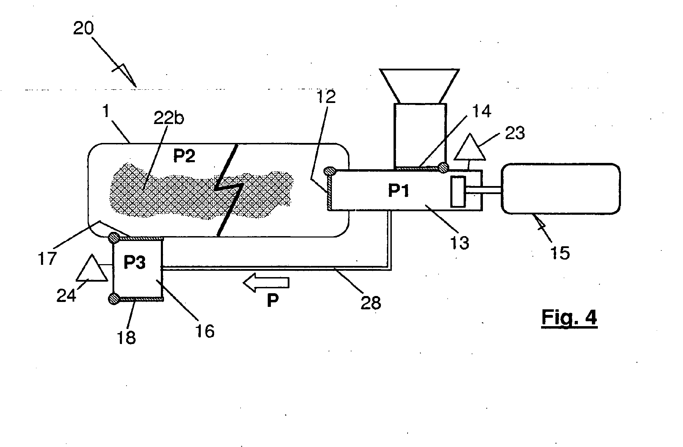

Once the waste 22b is inside the pressure tank 1 (situation represented in Fig. 4), the sluice gate 12 of the loading or compression chamber 13 is again closed and said loading or compression chamber 13 is depressurized until reaching the suitable pressure for the opening of its inlet gate 14 and a new cycle of waste is started with the introduction of a new batch of loaded waste 22a in the inlet or compression chamber 13.

We should mention that varied elements are usually found among the urban solid waste to treat which may have dimensions over those suitable to permit their intake either in the loading or compression chamber 13 or in the pressure tank 1, which may alter the behaviour of the sluice gates 12 and 14, hindering its subsequent extraction from the pressure tank 1 (for example, in the case of elongated and metal bodies which are not decomposed inside the pressure tank 1) or even produce damages therein. In order to avoid these drawbacks, before the operation of introducing a batch of loaded waste 22a in the loading or compression chamber 13, the waste of said batch of loaded waste 22a is sufficiently compacted to deform or break elements that exceed predetermined dimensions or to detect the inclusion of unbreakable elements that exceed said predetermined dimensions. In this last case, the batch of loaded waste 22a is temporary rejected and it is separated to be examined, manually or automatically extracting the unbreakable elements, and being later introduced in the inlet or compression chamber 13. In any case, it is not desirable that liquids contained in the container drip during the compacting operation.

In the example of Fig. 1, the means to transport 21, turn and/or agitate the waste 22b introduced from the inlet 30 to the outlet 29, comprises a rotary drum 25, coaxial with respect to the pressure tank 1, having perforated walls 27 and equipped with elements 26 which are projected from the inner surface of the side wall of said rotary drum 25. These projected elements 26 are designed to turn and move in longitudinal direction, respectively, the waste 22b introduced in said rotary drum with rotation around its longitudinal axis.

As has been represented in Fig. 1, the perforated wall 27 of the rotary drum 25 covers only an end longitudinal portion of said rotary drum 25, corresponding to the reception end of the batch of loaded waste 22a. And unlike conventional apparatus for waste treatment, the waste contained in a batch of loaded waste 22a introduced in the pressure tank 1 is instantaneously subjected to the temperature conditions inside the pressure tank 1 causing, among other effects, that the liquids contained in the waste are released at the start of their path through the rotary drum 25, in other words in the area close to the intake end of the pressure tank 1.

The function of the perforations is to evacuate the water for the interior of rotary drum 25, preventing the waste from flowing towards the exterior of the rotary drum. With the aim of retaining the waste inside the rotary drum, the diameter of the perforations are less than 7 mm, and preferabily are from 1 mm to 4 mm.

Regarding the gap between the perforations, it is from aproximately 5 mm to aproximately 20 mm.

It is also observed in Fig. 1 that the pressure tank 1 is disposed inclined with respect to the horizontal although, unlike the known apparatus, it is the end with the outlet 29 which is raised with respect to the end equipped with the inlet 30. In this way, the steam condensates and the liquids contained in the waste 22b which are precipitated in the pressure tank 1 are extracted therefrom through a connection 2 located in its lower part, in this case the intake end, using the interior overpressure and the inclination of the pressure tank 1. For said purpose, the apparatus 20 for waste treatment comprises a liquid level meter in charge of transmitting an activation signal to a central control system which regulates the extraction of liquids through the connection 2 when the level of liquid accumulated inside the tank exceeds a certain value.

In order to avoid the stopping up of the orifices of the perforated wall 27, the apparatus 20 is equipped with a series of sprinklers 5 designed to inject pressurized steam in a controlled manner against the outer wall of the rotary drum 25, and in particular directed against the portion provided with orifices to give the water free passage through it.

The retention time of the waste 22b inside the pressure tank 1 is determined by the length of the rotary drum 25 and its rotation rate. These parameters are adjusted so that the retention time of the waste, generally between 15 and 20 minutes inside the pressure tank 1 and subjected to the constant pressure and temperature conditions inside, is necessary for its correct sanitation.

Once the waste 22b has longitudinally travelled through the rotary drum 25, and, therefore, has remained therein the required time for its correct treatment, it should be extracted from the pressure tank 1. Thus, and in a similar way to the loading or compression chamber 13, the apparatus 20 comprises a discharge or decompression chamber 16 designed to house a batch of discharged waste 22c treated, equipped with separate inlet 17a and outlet channels 18a of the already treated waste, in this case the inlet channel 17a being juxtaposed to the outlet 29 of the pressure tank, which are provided with respective sluice gates 17 and 18.

In collaboration with the inlet 17a and outlet channels 18a, the apparatus 20 comprises means 24 to decrease the pressure inside the discharge or decompression chamber 16 to atmospheric pressure and thus allow the treated batch of discharged waste 22c to be expulsed outside without causing considerable depressurizations or temperature losses inside the pressure tank 1.

Due to the fact that the outlet 29 of the pressure tank 1 is disposed on the lower side thereof and that the inlet channel 17a of the discharge or decompression chamber 16 is disposed juxtaposed under said outlet 29, the waste 22b extracted from the pressure tank 1 is introduced in said discharge or decompression chamber 16 by gravity. Fig. 1 represents the sluice gate 17 of the discharge or decompression chamber 16 open, with the corresponding sluice gate 18 closed, so that batch of discharged waste 22c discharged by the rotary drum 25 is deposited in said discharge or decompression chamber 16.

To extract a treated batch of discharged waste 22c from inside the pressure tank 1 the following is carried out. Once the waste 22b has been deposited in the discharge or decompression chamber 16, the sluice gate 17 of said discharge or decompression chamber 16 closes (situation represented in Fig. 2) and the discharge or decompression chamber 16 is depressurized until the pressure P3 therein approaches or is equal to the pressure outside the apparatus. Next, the sluice gate 18 opens expulsing the batch of discharged waste 22c outside (situation represented in Fig. 3) which falls by gravity, the sluice gate 18 again closing (situation represented in Fig. 4) to start a new discharge cycle of the treated batch of discharged waste 22c which starts increasing the pressure P3 inside the discharge or decompression chamber 16 until the value of the pressure P2 inside the pressure tank 1 is reached.

As is shown in Figs. 2, 3 and 4, the apparatus 20 for waste treatment may be equipped with means of communication 28 between the loading or compression 13 and discharge or decompression chambers 16, whereby the internal pressures of both chambers can be equalled.

Thus, in Fig. 2 the batch of loaded waste 22a to treat is found in the loading or compression chamber 13 at a pressure P1 lower than the pressure P2 inside the pressure tank, which makes it necessary to increase the pressure P1 until reaching the value of the pressure P2 to introduce the waste in the pressure tank 1 without significantly altering the pressure conditions inside said pressure tank 1. At the same time, the already treated batch of discharged waste 22c has just come out of the inside of the pressure tank 1, which means the value of the pressure P3, equal to the pressure P2 inside the pressure tank 1, is higher than the outside or atmospheric pressure necessary to be able to expulse said batch of discharged waste 22c to the outside in a controlled and safe manner. Through the means of communication 28, the pressures P1 in the loading or compression chamber 13 and P3 in the discharge or decompression chamber 16 are equalled, i.e. the excess pressure from the depressurization of the batch of discharged waste 22c contributes to the increase in pressure of the batch of loaded waste 22a.

Similarly, in the situation represented in Fig. 4, the pressure P1 in the loading or compression chamber 13, previously in communication with the inside of the pressure tank 1, is equal to the pressure P2 inside said pressure tank 1, which is higher than the outside or atmospheric pressure at which the batch of loaded waste 22a to treat is found. Thus, before admitting a new batch of loaded waste 22a in the loading or compression chamber 13 this should be depressurized. At the same time, the discharge or decompression chamber 16 has just expulsed the batch of discharged waste 22c for which reason it is found at a pressure P3 close to the outside or atmospheric pressure. To be able to again receive the waste 22b from the inside of the pressure tank 1 without altering the pressure conditions inside said pressure tank 1, it will be necessary that the pressure P3 inside the discharge or decompression chamber 16 increases until reaching the value of the pressure P2 inside the pressure tank. Through the means of communication 28, the excess pressure from the depressurization of the loading or compression chamber 13 will contribute to the pressurization of the discharge or decompression chamber 16 necessary to admit the batch of discharged waste 22c therein.

Throughout the waste treatment process, the pressure and temperature conditions inside the pressure tank 1 are essentially maintained constant, it only being necessary to compensate the small pressure and temperature losses therein as a consequence of the operations of introduction and extraction of the waste inside it, but in no case being necessary to raise the pressure and the temperature from the ambient conditions outside the apparatus each time new waste is introduced in the pressure tank 1.

## Claims

1. Process for treating urban solid waste and similar industrial waste, that comprises the continuous operation of
- treating the waste (22b) in a pressure tank (1) provided with an inlet (30) and an outlet (29), while transporting said waste from the inlet to the outlet of the pressure tank, turning it and mixing said waste and subjecting said waste in an uninterrupted manner during its retention in the pressure tank to a pressure over 2 bar and a temperature between 100°C and 200°C,
and further comprising the discontinuous operations of
- subjecting to pressure, one by one, successive batches of loaded waste (22a) in a loading or compression chamber (13) from ambient pressure to that of inside the pressure tank and introducing them, also one by one, inside the pressure tank; and
- extracting from the pressure tank, also one by one, several already treated batches of discharged waste (22c) into a discharge or decompression chamber (16), said discharge or decompression chamber having previously reached the pressure of the pressure tank and
- depressurizing in the discharge or decompression chamber, also one by one, the several already treated batches of discharged waste (22c);
performing the corresponding compression and decompression operations in the respective loading or compression chamber and discharge or decompression chamber provided with pressure adjustment means,
wherein the pressure to which the waste is subjected is increased from ambient pressure to that of inside the pressure tank and vice-versa, respectively, compensating the small pressure and temperature losses inside the pressure tank, whose values are maintained essentially constant, after the waste introductions and extraction operations, **characterized in that** a new discharge cycle of the treated waste (22c) starts with increasing the pressure inside the discharge or decompression chamber (16) until the value of the pressure inside the discharge or decompression chamber (16) has reached the pressure in the pressure tank (1).

2. Process according to claim 1, **characterized in that** the waste (22b) is transported from the inlet (30) to the outlet (29) of the pressure tank (1) turning and mixing said waste through an interior rotary drum (25) coaxial with the pressure tank.

3. Process according to any of the preceding claims, **characterized in that** when a batch of loaded waste (22a) is placed inside the pressure tank (1), to be transported from the inlet (30) to the outlet (29) of the pressure tank, said batch of loaded waste (22a) initially passes through a first portion of the rotary drum (25) having perforated walls (27), the liquid flowing from the waste loading flowing towards the exterior of the rotary drum through the perforated walls.

4. Process according to the preceding claim, **characterized in that** the liquid flowing from the waste (22b) flowing towards the exterior of the rotary drum through the perforated walls is extracted through a connection (2) located in the lower part of the pressure tank (1) using the interior overpressure and the inclination of said pressure tank.

5. Process according to any of the claims 3 to 4, **characterized in that** while the batch of loaded waste (22a) passes through a first portion of the rotary drum (25) having perforated walls (27), pressurized steam is injected directed towards the outer wall of said first portion of the rotary drum (25).

6. Process according to any of the claims 3 to 5, **characterised in that** after the waste (22b) has passed through the first portion of the rotary drum (25) having perforated walls (27), said waste (22b) passes through a second portion of the rotatory drum not having perforated walls until being discharged outside of the rotatory drum towards the outlet (29) of the pressure tank (1).

7. Process according to the preceding claims, **characterized in that** the loading or compression chamber (13) and the discharge or decompression chamber (16) are communicated to transfer and equal the pressures in both chambers, before the compression or decompression operations of the chambers.

8. Process according to the preceding claim, **characterized in that** while one of the chambers (13,16) is performing a compression or decompression operation, the other chamber is performing the opposite operation.

9. Apparatus (20) for waste treatment, particularly urban solid waste (22b) and similar industrial waste, equipped with a fixed pressure tank (1) provided with means to raise and control the temperature to between 100ºC and 200ºC and means to raise the pressure over 2 bars in said pressure tank, said fixed pressure tank being arranged at one longitudinal end (4a) an inlet (30) of the waste to treat and an outlet (29) at the other longitudinal end (4b) of the treated waste, being disposed inside the pressure tank means to transport (21) the waste introduced from the inlet to the outlet of the pressure tank comprising:
- a loading or compression chamber (13) designed to house a batch of loaded waste (22a), equipped with separate inlet (14a) and outlet channels (12a) of the waste of which the outlet channel gives access, through the inlet (30) of the pressure tank, to inside said pressure tank,;
- a discharge or decompression chamber (16) designed to house a batch of unloaded waste (22c), equipped with separate inlet (17a) and outlet channels (18a) of the already treated waste, of which the inlet channel is juxtaposed to the outlet (29) of the pressure tank,;
- means (23) to increase and decrease the pressure inside the loading or compression chamber (13) from atmospheric pressure until reaching the value of the pressure inside the pressure tank and vice-versa;
- means (24) to decrease the pressure inside the discharge or decompression chamber (16) until reaching atmospheric pressure;
wherein
the means to transport the waste turn and agitate said waste, and
both inlet and outlet channels of the loading or compression chamber are being provided with respective gates (12; 14) and also both inlet and outlet channels of the discharge or decompression chamber are being provided with respective gates (17; 18), **characterized in that**
said means (24) to decrease the pressure inside the discharge or decompression chamber (16) also allow to increase the pressure inside said discharge or decompression chamber from atmospheric pressure until reaching the value of the pressure inside the pressure tank and that said gates (12; 14; 17; 18) are sluice gates.

10. Apparatus according to the preceding claim, **characterised in that** the means to transport (21), turn and agitate the waste (22b) inside the pressure tank (1) comprise a rotary drum (25), coaxial with respect to the pressure tank, of perforated walls (27) and equipped with elements (26) which are projected from the inner surface of the side wall of said rotary drum, designed to turn and to move in longitudinal direction, respectively, the waste introduced therein with the rotation of the rotary drum around its longitudinal axis.

11. Apparatus (20) according to the preceding claim, **characterised in that** the walls (27) of the rotary drum (25) are only perforated along an end longitudinal portion of said rotary drum, corresponding to the reception end of the waste and **in that** the pressure tank (1) is provided with sprinklers (5) arranged to inject pressurised steam directed against the perforated walls (27) of the rotary drum.

12. Apparatus (20) according to any of the preceding claims 9 to 11, **characterized in that** the diameter of the perforations are from 1 mm to 7 mm.

13. Apparatus (20) according to claims 9 to 12, **characterized in that** the outlet channel (12a) of the loading or compression chamber (13) is disposed inside the pressure tank (1) arranged so that, when the batch of loaded waste (22a) contained in said loading or compression chamber is extracted, it is deposited, by gravity, in the means to transport (21), turn and-agitate the waste; and **in that** the loading or compression chamber of the batch of loaded waste is essentially cylindrical and is disposed horizontally, and **in that** it is equipped with means of pushing (15) the waste contained in the direction of the outlet channel (12a).

14. Apparatus (20) according to the preceding claim, **characterized in that** the means of pushing (15) comprise a hydraulic cylinder which can move longitudinally through the inside of the loading or compression chamber (13).

15. Apparatus (20) according to the claims 9 to 14, **characterized in that** it is equipped with means of communication (28) between the loading or compression (13) and discharge or decompression chambers (16), whereby the internal pressures of both chambers can be equalled.

16. Apparatus (20) according to the claims 9 to 15, **characterized in that** the pressure tank (1) is disposed inclined with respect to the horizontal and so that the end equipped with the outlet (29) is raised with respect to the end equipped with the inlet (30).

## Patentansprüche

1. Verfahren zur Behandlung von festem Siedlungsabfall und ähnlichem Industrieabfall, umfassend den kontinuierlichen Vorgang der
- Behandlung des Abfalls (22b) in einem Druckbehälter (1), welcher mit einem Eingang (30) und mit einem Ausgang (29) versehen ist, während der genannte Abfall von dem Eingang bis zum Ausgang des Druckbehälters transportiert wird, unter Umwälzen und Mischen des genannten Abfalls und Aussetzen des genannten Abfalls auf eine ununterbrochene Weise während der Zurückhaltung desselben in dem Druckbehälter einem Druck von mehr als 2 Bar und einer Temperatur zwischen 100ºC und 200ºC,
und zusätzlich umfassend die unterbrochene Vorgänge der
- Druckaussetzung, einzeln, von sukzessiven Batches von geladenem Abfall (22a) in einer Lade- oder Kompressionskammer (13) von dem Umgebungsdruck auf den Druck innerhalb des Druckbehälters und die Einführung derselben, ebenso einzeln, in den Druckbehälter; und
- Entnahme aus dem Druckbehälter, ebenso einzeln, von mehreren schon behandelten Batches von entladenem Abfall (22c) in eine Entlade- oder Dekompressionskammer (16), wobei die genannte Entlade- oder Dekompressionskammer vorher den Druck des Druckbehälters erreicht hat und
- Herabsetzen des Drucks in der Entlade- oder Dekompressionskammer, ebenso einzeln, von den mehreren schon behandelten Batches von entladenem Abfall (22c);
unter Ausführung der entsprechenden Kompressions- und Dekompressionsvorgänge in der jeweiligen Lade- oder Kompressionskammer und Entlade- oder Dekompressionskammer, die mit Druckeinstellungsmitteln versehen sind,
wobei der Druck, welchem der Abfall ausgesetzt wird, von dem Umgebungsdruck auf den Druck innerhalb des Druckbehälters erhöht wird und umgekehrt, wobei jeweils die kleinen Druck- und Temperaturverluste innerhalb des Druckbehälters ausgeglichen werden, wobei die Werte derselben nach den Vorgängen der Abfalleinführung und -entnahme wesentlich konstant gehalten werden, **dadurch gekennzeichnet, dass** ein neues Entladezyklus des behandelten Abfalls (22c) mit der Erhöhung des Drucks innerhalb der Entlade- oder Dekompressionskammer (16) beginnt, bis der Wert des Drucks innerhalb der Entlade- oder Dekompressionskammer (16) den Druck in dem Druckbehälter (1) erreicht hat.

2. Verfahren nach Anspruch 1, **dadurch gekennzeichnet, dass** der Abfall (22b) von dem Eingang (30) bis zum Ausgang (29) des Druckbehälters (1) transportiert wird, wobei der genannte Abfall durch eine innere Drehtrommel (25), die mit dem Druckbehälter koaxial ist, umgewälzt und gemischt wird.

3. Verfahren nach einem der vorhergehenden Ansprüche, **dadurch gekennzeichnet, dass**, wenn ein Batch von geladenem Abfall (22a) innerhalb des Druckbehälters (1) gelegt wird, um von dem Eingang (30) bis zum Ausgang (29) des Druckbehälters transportiert zu werden, das genannte Batch von geladenem Abfall (22a) anfangs durch einen ersten Teil der Drehtrommel (25), der durchlochte Wände (27) aufweist, läuft, wobei die Flüssigkeit, die aus der Abfallladung fließt, zum äußeren Teil der Drehtrommel durch die durchlochten Wände fließt.

4. Verfahren nach dem vorhergehenden Anspruch, **dadurch gekennzeichnet, dass** die Flüssigkeit, die aus dem Abfall (22b) fließt, die zum äußeren Teil der Drehtrommel durch die durchlochten Wände fließt, durch einen Anschluss (2), der sich in dem unteren Teil des Druckbehälters (1) befindet, unter Verwendung des inneren Überdrucks und der Neigung des genannten Druckbehälters, entnommen wird.

5. Verfahren nach einem der Ansprüche 3 bis 4, **dadurch gekennzeichnet, dass**, während das Batch von geladenem Abfall (22a) durch einen ersten Teil der Drehtrommel (25), der durchlochte Wände (27) aufweist, läuft, unter Druck stehender Dampf, welcher zur Außenwand des ersten Teils der Drehtrommel (25) gerichtet wird, eingespritzt wird.

6. Verfahren nach einem der Ansprüche 3 bis 5, **dadurch gekennzeichnet, dass**, nachdem der Abfall (22b) durch den ersten Teil der Drehtrommel (25), der durchlochte Wände (27) aufweist, gelaufen ist, der genannte Abfall (22b) durch einen zweiten Teil der Drehtrommel läuft, der keine durchlochte Wände aufweist, bis er außerhalb der Drehtrommel zum Ausgang (29) des Druckbehälters (1) entladen wird.

7. Verfahren nach den vorhergehenden Ansprüche, **dadurch gekennzeichnet, dass** die Lade- oder Kompressionskammer (13) und die Entlade- oder Dekompressionskammer (16) miteinander kommunizieren, um die Drücke in beiden Kammern, vor den Kompressions- und Dekompressionsvorgängen der Kammern, zu übertragen und auszugleichen.

8. Verfahren nach dem vorhergehenden Anspruch, **dadurch gekennzeichnet, dass**, während eine der Kammern (13, 16) einen Kompressions- oder Dekompressionsvorgang ausführt, die andere Kammer den entgegengesetzten Vorgang ausführt.

9. Vorrichtung (20) zur Abfallbehandlung, insbesondere von festem Siedlungsabfall (22b) und ähnlichem Industrieabfall, ausgerüstet mit einem festen Druckbehälter (1), der mit Mitteln für die Erhöhung und Regelung der Temperatur auf zwischen 100ºC und 200ºC und mit Mitteln für die Erhöhung des Drucks auf über 2 Bar in dem genannten Druckbehälter versehen ist, wobei in dem genannten festen Druckbehälter an einem longitudinalen Ende (4a) ein Eingang (30) für den zu behandelnden Abfall und an dem anderen longitudinalen Ende (4b) ein Ausgang (29) für den behandelten Abfall angeordnet sind, wobei innerhalb des Druckbehälters Mittel (21) für den Transport des Abfalls, der von dem Eingang aus zum Ausgang des Druckbehälters eingeführt wird, angeordnet sind, umfassend
- eine Lade- oder Kompressionskammer (13), welche dazu ausgebildet ist, ein Batch von geladenem Abfall (22a) aufzunehmen, die mit einem Eingangskanal (14a) und einem Ausgangskanal (12a) für den Abfall, die getrennt sind, ausgerüstet ist, wobei der Ausgangskanal, durch den Eingang (30) des Druckbehälters, Zugang zum Inneren des genannten Druckbehälters ermöglicht;
- eine Entlade- oder Dekompressionskammer (16), welche dazu ausgebildet ist, ein Batch von entladenem Abfall (22c) aufzunehmen, die mit einem Eingangskanal (17a) und einem Ausgangskanal (18a) für den schon behandelten Abfall, die getrennt sind, ausgerüstet ist, wobei der Eingangskanal neben dem Ausgang (29) des Druckbehälters gestellt ist;
- Mittel (23) für die Erhöhung und Verringerung des Drucks innerhalb der Lade- oder Kompressionskammer (13) von dem Umgebungsdruck bis der Wert des Drucks innerhalb des Druckbehälters, und umgekehrt, erreicht wird;
- Mittel (24) für die Verringerung des Drucks innerhalb der Entlade- oder Dekompressionskammer (16) bis der Umgebungsdruck erreicht wird;
wobei
die Mittel für den Transport des Abfalls den genannten Abfall umwälzen und schütten, und
wobei sowohl der Eingangskanal als auch der Ausgangskanal der Lade- oder Kompressionskammer mit jeweiligen Toren (12; 14) versehen sind und sowohl der Eingangskanal als auch der Ausgangskanal der Entlade- oder Dekompressionskammer mit jeweiligen Toren (17; 18) versehen sind; **dadurch gekennzeichnet, dass** die genannten Mittel (24) für die Verringerung des Drucks innerhalb der Entlade- oder Dekompressionskammer (16) auch die Erhöhung des Drucks innerhalb der genannten Entlade- oder Dekompressionskammer von dem Umgebungsdruck bis der Wert des Drucks innerhalb des Druckbehälters erreicht wird, ermöglichen, und dass die genannten Tore (12; 14; 17; 18) Schleusentore sind.

10. Vorrichtung nach dem vorhergehenden Anspruch, **dadurch gekennzeichnet, dass** die Mittel (21) für den Transport, die Umwälzung und das Schütteln des Abfalls (22b) innerhalb des Druckbehälters (1) eine Drehtrommel (25) mit durchlochten Wänden (27), welche in Bezug auf den Druckbehälter koaxial ist, und welche mit Elementen (26) ausgerüstet ist, welche von der inneren Oberfläche der Seitenwand der genannten Drehtrommel hervorstehen, umfassen, wobei die Elemente (26) dazu ausgebildet sind, den in diesem eingeführten Abfall mit der Drehung der Drehtrommel um ihre Längsachse jeweils umzuwälzen und in der Längsrichtung zu bewegen.

11. Vorrichtung (20) nach dem vorhergehenden Anspruch, **dadurch gekennzeichnet, dass** die Wände (27) der Drehtrommel (25) nur entlang eines longitudinalen Endteils der genannten Drehtrommel, entsprechend dem Aufnahmeende des Abfalls, durchlocht sind, und dass der Druckbehälter (1) mit Sprinklern (5) versehen ist, die für die Einspritzung von unter Druck stehendem Dampf angeordnet sind, wobei der unter Druck stehende Dampf gegen die durchlochte Wände (27) der Drehtrommel gerichtet wird.

12. Vorrichtung (20) nach einem der vorhergehenden Ansprüche 9 bis 11, **dadurch gekennzeichnet, dass** der Durchmesser der Löcher von 1 mm bis 7 mm ist.

13. Vorrichtung (20) nach den Ansprüchen 9 bis 12, **dadurch gekennzeichnet, dass** der Ausgangskanal (12a) der Lade- oder Kompressionskammer (13) innerhalb des Druckbehälters (1) angeordnet ist, so dass wenn das Batch von geladenem Abfall (22a), welches in der genannten Lade- oder Kompressionskammer enthalten ist, entnommen wird, dieses durch die Schwerkraft in den Mitteln (21) für den Transport, die Umwälzung und das Schütteln des Abfalls deponiert wird; und dass die Lade- oder Kompressionskammer von dem Batch von geladenem Abfall wesentlich zylindrisch und horizontal angeordnet ist, und dass sie mit Mitteln (15) ausgerüstet ist, um den enthaltenen Abfall in Richtung des Ausgangskanals (12a) zu drücken.

14. Vorrichtung (20) nach dem vorhergehenden Anspruch, **dadurch gekennzeichnet, dass** die Mittel (15) zum Drücken einen Hydraulikzylinder umfassen, welcher sich durch das Innere der Lade- oder Kompressionskammer (13) longitudinal bewegen kann.

15. Vorrichtung (20) nach den Ansprüchen 9 bis 14, **dadurch gekennzeichnet, dass** sie mit Mitteln (28) für die Kommunikation zwischen der Lade- oder Kompressionskammer (13) und der Entlade- oder Dekompressionskammer (16) ausgerüstet ist, wodurch die inneren Drücke von beiden Kammern ausgeglichen werden können.

16. Vorrichtung (20) nach den Ansprüchen 9 bis 15, **dadurch gekennzeichnet, dass** der Druckbehälter (1) in Bezug auf die Horizontale geneigt angeordnet ist und so dass das Ende, welches mit dem Ausgang (29) ausgerüstet ist, in Bezug auf das Ende, welches mit dem Eingang (30) ausgerüstet ist, gehoben ist.

## Revendications

1. Procédé pour traiter des déchets urbains solides et des déchets industriels similaires, comprenant l'opération continue de
- traiter les déchets (22b) dans un réservoir pressurisé (1) pourvu d'une entrée (30) et d'une sortie (29), tout en transportant lesdits déchets depuis l'entrée jusqu'à la sortie du réservoir pressurisé, tourner et mélanger lesdits déchets et soumettre lesdits déchets de façon ininterrompu durant sa rétention dans le réservoir pressurisé à une pression supérieure à 2 bar et à une température entre 100ºC et 200ºC,
et comprenant en outre les opérations discontinues de
- soumettre à pression, un par un, de façon successive des lots de déchets chargés (22a) dans une chambre de chargement ou de compression (13) à partir de la pression ambiante jusqu'à celle de l'intérieur du réservoir pressurisé et les introduire, aussi un par un, à l'intérieur du réservoir pressurisé; et
- extraire du réservoir pressurisé, aussi un par un, plusieurs lots déjà traités de déchets déchargés (22c) dans une chambre de déchargement ou de décompression (16), ladite chambre de déchargement ou de décompression ayant atteinte préalablement la pression du réservoir pressurisé et
- dépressuriser dans la chambre de déchargement ou de décompression, aussi un par un, les multiples lots déjà traités de déchets déchargés (22c);
performer les opérations de compression et décompression correspondantes dans leurs respectives chambre de chargement ou de compression et chambre de déchargement ou de décompression pourvues de moyens d'ajustement de pression
dans lequel la pression à laquelle les déchets sont soumis est augmentée depuis la pression ambiante jusqu'à celle de l'intérieur du réservoir pressurisé et vice et versa, respectivement, compensant les petites pertes de pression et de température à l'intérieur du réservoir pressurisé, dont les valeurs sont maintenus essentiellement constantes, après les opérations d'introduction et d'extraction des déchets, **caractérisé en ce qu'**un nouveau cycle de déchargement des déchets traités (22c) commence avec une augmentation de la pression à l'intérieur de la chambre de déchargement ou de décompression (16) jusqu'à ce que la valeur de la pression à l'intérieur de la chambre de déchargement ou de décompression (16) ait atteint la pression à l'intérieur du réservoir pressurisé (1).

2. Procédé selon la revendication 1, **caractérisé en ce que** les déchets (22b) sont transportés depuis l'entrée (30) jusqu'à la sortie (29) du réservoir pressurisé (1) en tournant et mélangeant lesdits déchets au travers d'un tambour rotatif intérieur (25) coaxial avec le réservoir pressurisé.

3. Procédé selon l'une quelconque des revendications précédentes, **caractérisé en ce que** lorsqu'un lot de déchets chargés (22a) est placé à l'intérieur du réservoir pressurisé (1), pour être transporté depuis l'entrée (30) jusqu'à la sortie (29) du réservoir pressurisé, lesdits lots de déchets chargés (22a) au début passe au travers d'une première portion du tambour rotatif (25) ayant des parois perforées (27), le liquide coulant depuis les chargement de déchets coulant vers l'extérieur du tambour rotatif au travers des parois perforées.

4. Procédé selon la revendication précédente, **caractérisé en ce que** le liquide coulant depuis les déchets (22b) coulant vers l'extérieur du tambour rotatif au travers des parois perforées est extrait au travers d'une connexion (2) situé dans une partie inferieur du réservoir pressurisé (1) utilisant la surpression interne et l'inclination dudit réservoir pressurisé.

5. Procédé selon l'une quelconque des revendications 3 à 4, **caractérisé en ce que** pendant que le lot de déchets chargés (22a) passe au travers d'une première portion du tambour rotatif (25) ayant des parois perforées (27), de la vapeur pressurisée est injecté en direction de la paroi extérieure de ladite première portion du tambour rotatif (25).

6. Procédé selon l'une quelconque des revendications 3 à 5, **caractérisé en ce qu'**après que les déchets (22b) ont passés au travers de la première portion du tambour rotatif (25) ayant des parois perforées (27), lesdits déchets (22b) passent au travers d'une deuxième portion du tambour rotatif n'ayant pas des parois perforées jusqu'à être déchargés en dehors du tambour rotatif vers la sortie (29) du réservoir pressurisé (1).

7. Procédé selon les revendications précédentes, **caractérisé en ce que** la chambre de chargement ou de compression (13) et la chambre de déchargement ou de décompression (16) sont reliées pour transférer et égaliser les pressions dans les deux chambres, avant les opérations de compression ou de décompression des chambres.

8. Procédé selon la revendication précédente, **caractérisé en ce que** pendant qu'une des chambres (13, 16) est en train de performer une opération de compression ou décompression, l'autre chambre performe l'opération opposé.

9. Dispositif (20) pour le traitement des déchets, particulièrement des déchets urbains solides (22b) et des déchets industriels similaires, équipé avec un réservoir pressurisé fixe (1) pourvu de moyens pour augmenter et pour contrôler la température entre 100ºC et 200ºC et de moyens pour augmenter la pression au dessus de 2 bar dans ledit réservoir pressurisé, dans ledit réservoir pressurisé fixe étant arrangées sur un bout longitudinal (4a) une entrée (30) des déchets à traiter et une sortie (29) sur l'autre bout longitudinal (4b) des déchets traités, à l'intérieur du réservoir pressurisé étant disposés des moyens pour transporter (21) les déchets introduits depuis l'entrée jusqu'à la sortie du réservoir pressurisé comprenant:
- une chambre de chargement ou de compression (13) désignée pour loger un lot de déchets chargés (22b), équipée d'une entrée séparée (14a) et de canaux de sortie (12a) de déchets desquelles le canal de sortie donne accès, au travers de l'entrée (30) du réservoir pressurisé, à l'intérieur dudit réservoir pressurisé;
- une chambre de déchargement ou de décompression (16) désignée pour loger un lot de déchets déchargés (22c), équipée d'une entrée séparée (17a) et de canaux de sortie (18a) de déchets déjà traités, desquelles le canal d'entrée est juxtaposé à la sortie (29) du réservoir pressurisé ;
- des moyens (23) pour augmenter et diminuer la pression à l'intérieur de la chambre de chargement ou de compression (13) à partir de la pression atmosphérique jusqu'à atteindre la valeur de la pression à l'intérieur du réservoir pressurisé et vice versa;
- des moyens (24) pour réduire la pression à l'intérieur de la chambre de déchargement ou de décompression (16) jusqu'à atteindre la pression atmosphérique;
dans lequel
les moyen pour transporter les déchets tournent et agitent lesdits déchets, et,
les deux canaux d'entrée et de sortie de la chambre de chargement ou de compression sont pourvus de portes respectives (12; 14) et aussi les deux canaux d'entrée et de sortie de la chambre de déchargement ou de décompression sont pourvus de portes respectives (17; 18); **caractérisé en ce que**
lesdits moyens (24) pour réduire la pression à l'intérieur de la chambre de déchargement ou de décompression (16) permettent aussi d'augmenter la pression à l'intérieur de ladite chambre de déchargement ou de décompression à partir de la pression atmosphérique jusqu'à atteindre la valeur de la pression à l'intérieur du réservoir pressurisé et **en ce que** lesdites portes (12; 14; 17; 18) sont des portes d'écluse.

10. Dispositif selon la revendication précédente, **caractérisé en ce que** les moyens pour transporter (21) tournent et agitent les déchets (22b) à l'intérieur du réservoir pressurisé (1) comprenant un tambour rotatif (25), coaxial par rapport au réservoir pressurisé, des parois perforées (27) et équipé avec des éléments (26) qui font saillie depuis la surface interne de la paroi latérale dudit tambour rotatif, désigné pour tourner et bouger dans une direction longitudinal, respectivement, les déchets introduits dans celui-ci avec la rotation du tambour rotatif autour de son axe longitudinal.

11. Dispositif (20) selon la revendication précédente, en ce que les parois (27) du tambour rotatif (25) sont seulement perforées au long d'une portion longitudinale de bout dudit tambour rotatif, correspondant au bout recevant les déchets et en que ce le réservoir pressurisé (1) est pourvu des asperseurs (5) arrangés pour injecter de la vapeur pressurisée dirigée contre les parois perforées (27) du tambour rotatif.

12. Dispositif (20) selon l'une quelconque des revendications précédentes 9 à 11, **caractérisé en ce que** le diamètre des perforations est de 1 mm jusqu'à 7 mm.

13. Dispositif (20) selon les revendications 9 à 12, **caractérisé en ce que** le canal de sortie (12a) de la chambre de chargement ou de compression (13) est disposé à l'intérieur du réservoir pressurisé (1) arrangé de façon à ce que lorsque le lot de déchets chargés (22a) contenu dans ladite chambre de chargement ou de compression est extrait, il est déposé, par gravité, dans les moyens pour transporter (21), tourner et agiter les déchets; et **en ce que** la chambre de chargement ou de compression du lot de déchets chargés est essentiellement cylindrique et est déposée horizontalement, et **en ce qu'**il est équipé de moyens de poussage (15) des déchets contenus en direction du canal de sortie (12a).

14. Dispositif (20) selon la revendication précédente, **caractérisé en ce que** les moyens de poussage (15) comprennent un cylindre hydraulique qui peut se déplacer longitudinalement au travers de l'intérieur de la chambre de chargement ou de compression (13).

15. Dispositif (20) selon les revendications 9 à 14, **caractérisé en ce qu'**il est équipé de moyens de communication (28) entre la chambre de chargement ou de compression (13) et la chambre de déchargement ou de décompression (16), selon lequel les pressions internes des deux chambres peuvent être égalées.

16. Dispositif (20) selon les revendications 9 à 15, **caractérisé en ce que** le réservoir pressurisé (1) est disposé de façon inclinée par rapport à l'horizontal et de façon à ce que le bout équipé avec la sortie (29) est élevé par rapport au bout équipé avec l'entrée (30).
